(19) Europäisches Patentamt
European Patent Office
Office européen des brevets

(11) Publication number: 0 145 875 B1

(12) # EUROPEAN PATENT SPECIFICATION

(45) Date of publication of patent specification: 19.02.92

(51) Int. Cl.5: C07D 307/30, C07D 333/36, C07D 333/40, //A61K31/34, A61K31/38

(21) Application number: 84112117.1

(22) Date of filing: 10.10.84

(54) **Gamma-aminobutyric acid transaminase inhibitors and processes for their preparation.**

(30) Priority: 11.10.83 US 540744
11.10.83 US 540743
11.10.83 US 540745

(43) Date of publication of application:
26.06.85 Bulletin 85/26

(45) Publication of the grant of the patent:
19.02.92 Bulletin 92/08

(84) Designated Contracting States:
AT BE CH DE FR GB IT LI LU NL SE

(56) References cited:
US-A- 3 983 134

AUSTRALIAN JOURNAL OF CHEMISTRY, vol. 37, no. 5, 1984, pages 1123-1126; R.D. ALLAN et al.: "Synthesis of analogues of GABA, XII* cis- and trans- 4-aminotetrahydrofuran-2-carboxylic acid"

(73) Proprietor: MERRELL DOW PHARMACEUTICALS INC.
2110 East Galbraith Road
Cincinnati Ohio 45215-6300(US)

(72) Inventor: Adams, Jerry L.
853 Delmont Avenue
Winnewood Pennsylvania 19096(US)
Inventor: Holbert, Gene W.
266 Stockton Drive
Loveland Ohio 45140(US)
Inventor: Metcalf, Brian W.
520 Woodland Drive
Radnor Pennsylvania 19087(US)
Inventor: Burkhart, Joseph P.
7290 Barrett Road
West Chester Ohio 45069(US)
Inventor: Lippert, Bruce J.
32 Fleming Road
Cincinnati Ohio 45215(US)

(74) Representative: Sgarbi, Renato et al
GRUPPO LEPETIT S.p.A. Patent and Trademark Department Via Roberto Lepetit, 34
I-21040 Gerenzano (Varese)(IT)

EP 0 145 875 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to 4-amino-4,5-dihydro-2-furancarboxylic acid, and 4-amino-4,5-dihydro-2 thiophenecarboxylic acids, the lower alkyl esters thereof and the pharmaceutically acceptable salts thereof, to their use as chemotherapeutic agents, and to the chemical processes and intermediates useful in the preparation thereof.

More specifically, this invention relates to $\gamma$-aminobutyric acid transaminase inhibitors useful in the treatment of epilepsy. These inhibitors are of the formula I

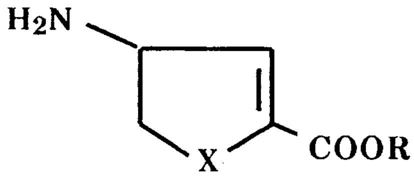

I

including the individual enantiomers and racemic mixtures thereof, and the pharmaceutically acceptable salts thereof, wherein R is hydrogen or a ($C_1$-$C_6$) lower alkyl radical, and X is oxygen or sulfur.

US.A.398 3134 describes some 3-amino-pyrrole or thiophene derivatives as intermediates for the preparation of wreylene thiophane compounds.

The preparation of the compounds of this invention may be achieved by the judicial selection of the appropriate starting materials followed by the application of chemical reactions and techniques analogously known in the art.

The compounds of the invention can be prepared according to the following procedure:

a) when a compound of formula I wherein X is oxygen is desired, deprotecting an N-protected-4-amino-4,5-dihydro-2-furancarboxylic acid or ($C_1$-$C_6$) alkyl ester thereof by reaction under acidic conditions,

b) when a compound of formula I wherein X is sulfur is desired, deprotecting an N-protected-4-amino-4,5-dihydro-2-thiophenecarboxylic acid or ($C_1$-$C_6$) lower alkyl ester thereof by reation under acidic conditions,

c) when a compound of formula I wherein R is hydrogen is obtained according to step a) or b), optionally converting it into the corresponding ($C_1$-$C_6$)alkyl ester by esterification procedures; or, when a lower alkyl ester is obtained according to step a) or b), optionally converting it into another ($C_1$-$C_6$)lower alkyl ester by transesterification procedures.

A compound of formula I wherein X is oxygen, in a racemic form, is prepared by a process which comprises:

a) converting a ($C_1$-$C_6$)alkyl tetrahydro-3-iodo-4-isocyanato-2-furancarboxylate into the corresponding carbamate by reaction with an alcohol,

b) transforming the obtained ($C_1$-$C_6$)alkyl-tetrahydro-3-iodo-4-carbamate-2-furancarboxylate into the corresponding ($C_1$-$C_6$)alkyl-4,5-dihydro-4-carbamate-2-furancarboxylate by reacting it with diazabicyclooctane in acetone,

c) hydrolyzing the alkyl ester bond to give the corresponding free acid, and

d) submitting the thus obtained 4,5-dihydro-4-carbamate-2-furancarboxylic acid to acid hydrolysis to give the corresponding amine derivative of formula I.

One such process for preparing the (R), (S) or (RS) racemic mixtures of 4-amino-4,5-dihydro-2-furancarboxylic acid is by employing the selected optical isomer, or racemic mixture thereof, of arabinose as a starting material.In each instance the chemical reaction of the sequential series of steps of chemical reactions would be the same but for the stereoisomers, or mixture produced. For example, starting with L-arabinose that optical isomeric form would be maintained throughout the process to finally produce the (S) enantiomer of the desired product. Similarly, starting with D-arabinose will ultimately produce the (R) enantiomer. Thus, although throughout the description and specific exemplification of the process for preparing the 4-amino-4,5-dihydro-2-furancarboxylic acid and esters of formula I from arabinose the L isomer is utilized, it is to be understood that such description and exemplification embraces the obtention of both the optical isomers and the mixtures thereof.

In the process starting with L-arabinose it is convenient to convert arabinose to a triol according to the method described for the D-isomer by Rabinson and Fletcher, (J.O.C. 32, 3452, 1968). The so-obtained triol is selectively silylated to protect the 2-position primary alcohol by standard reaction conditions utilizing t-butyl dimethylsilyl chloride in the presence of triethylamine and 4-dimethylaminopyridine, the reaction being

effected at room temperature. Following this selective silylation both the 3- and 4-position hydroxy groups are protected with mesylate leaving groups by standard reaction with mesylchloride to form a bismesylate.

Following the bis-mesylation, reaction of the 2-[[[(1,1-dimethylethyl)dimethylsilyl]oxy]methyl]tetrahydro-3,4-furandiol-bis-(methylsulfonate) with sodium azide will selectively displace the 4-position mesylate to form the desired azide. Following that step the 2-position primary alcohol is liberated by removal of the silyl protecting group and the resulting primary alcohol function is converted into the corresponding carboxylic acid function by oxidation, preferably by means of the Jones reagent (a solution of chromic acid and sulfuric acid in water).

The obtained product, 4-azido-tetrahydro-3-[(methylsulfonyl)oxy]-2-furancarboxylate, is then submitted to esterification, preferably forming a methyl ester, although other esters may optionally be formed. Elimination of the remaining 3-position mesyl group by reaction with triethylamine at room temperature will form the desired 2,3-double bond and the resulting methyl-4-azido-4,5-dihydro-2-furancarboxylate is chemically reduced to the amine by reaction with triethylamine and propanethiol. Alternatively, the reduction of the azide may be effected with a hydrogen Lindlar catalyst using ethanol as a solvent. Following the reduction of the azide the ester is hydrolyzed by reaction with lithium hydroxide and methanol at room temperature.

A process which yields only the racemic mixture of the desired compounds of this invention (Formula I) advantageously utilizes 2-furoic acid as the starting material.

In the initial step the 2-furoic acid is subjected to chemical reduction to yield 2,5-dihydro-2-furoic acid by reaction with lithium and ammonia in absolute ethanol according to standard reductive conditions. The so-obtained furoic acid is esterified and the resulting ester (preferably the methyl ester), when subjected to silver isocyanate and iodine will yield, in situ,the desired methyl tetrahydro-3-iodo-4-isocyanato-2-furancarboxylate. The isocyanate function is converted to a carbamate derivative with an alcohol reactant, (preferably utilizing a p-methoxybenzyl alcohol reactant) to protect the amine function. This reaction is followed by elimination of HI by stirring the iodo compound with diazobicyclooctane in acetone, to form the corresponding 4,5-dihydro compound. Following the formation of the desired double bond the ester is saponified and the resulting compound is subjected to acid hydrolysis with trifluoroacetic acid in anisole under an inert atmosphere (argon) to convert the carbamate to the free amine.

Another process for preparing the 4-amino-4,5-dihydro-2-furancarboxylic acid compounds of this invention utilizes either the D or the L- forms of glutamic acid-$\gamma$-alkyl esters, preferably the methyl ester; the L-isomer producing the (S) form of 4-amino-4,5-dihydro-2-furancarboxylic acid while the D-form yields the (R) enantiomer. It should therefore be noted that in the description and illustrations of the process steps of this invention the enantiomeric form of the final product, as well as all of the intermediate compounds produced by the individual steps of this process, will be similar to that of the starting glutamic acid. In addition to its use in preparing either the R or the S enantiomers, the process may be adapted to the preparation of the racemic mixture by starting with the racemic mixture of the glutamic acid. Further, although the methyl ester of glutamic acid is utilized as the starting material, other lower alkyl esters may similarly be employed in the event such esters are desired as the final product.

Starting with the $\gamma$-methyl ester of the D-, L-, or the DL forms of glutamic acid, the amine moiety is protected by the formation of any of the known N-protecting groups by procedures well known in the art, although in practice it is preferred to utilize the t-butoxycarbonyl protective group which is prepared by reacting the ester with di-t-butyldicarbonate in the presence of a basic catalyst, preferably consisting of dioxane:water and triethylamine. The reaction is effected by contacting the reagents together under ice-bath conditions. The so-obtained N-protected compound is subjected to an anhydride formation and selective sodium borohydride reduction of the newly formed anhydride to its alcohol. The anhydride formation is performed with stoichiometric amounts of isobutylchloroformate, in the presence of triethylamine under vigorous stirring conditions whilst maintaining the temperature of the reaction mixture at cold temperatures and under an argon (or other inert gas) atmosphere. The sodium borohydride reduction is effected at temperatures less than 15 °C.

The hydroxy moiety of the so-obtained methyl 4-[[(1,1-dimethylethoxy)carbonyl]amino]-5-hydroxy-pentanoate is silylated by reaction with t-butyldimethylsilyl chloride under strongly basic conditions (using 4-dimethylaminopyridine and triethylamine) at low temperatures, preferably at about 0 °C. Utilizing freshly prepared lithium diisopropylamine under an inert atmosphere (argon) an enolate anion is formed (on the carbon atom adjacent the $CO_2CH_3$ ester group) and the so-formed enol is subjected to silylation with trimethylsilyl chloride to form a ketene acetal. (Concurrently therewith, the N-atom is also silylated). The silyl ketene acetal is then subjected to a strong oxidizing agent, (i.e., a mineral acid, preferably a per acid such as m-chloroperoxybenzoic acid) to epoxidize the olefin. Treatment of this product in methanol/water leads to methyl 4-[[(1,1-dimethylethoxy)carbonyl]amino]-5-[[(1,1-dimethylethyl)dimethylsilyl]oxy]-2-hydroxy-pentanoate which is oxidized with pyridinium dichromate to form an α-keto ester.

The α-keto ester is hydrolized with aqueous perchloric acid (or other equivalently functioning acids such as hydrochloric or sulfuric acids) to remove the silyl group and, in the process of its removal ring cyclization occurs to form a hemi-ketal, which ketal is dehydrated with thionyl chloride in the presence of pyridine to yield a methyl 4-[[(1,1-dimethylethoxy)carbonyl]amino]-4,5-dihydro-2-furancarboxylate. Although it is feasible to deprotect the amine function of the foregoing product(s) prior to hydrolysis of the ester group, it is preferred to convert the ester to its acid prior to deprotecting the amine and then, if desired, re-esterify the acid compound.

In effecting the hydrolysis it is preferred to utilize methanol in THF in the presence of aqueous lithium hydroxide, keeping the reaction mixture at ice-bath temperatures. The removal of the t-butoxycarbonyl protecting group is achieved by reacting the product of the previous step (preferably the acid form) with freshly prepared trifluoroacetic acid whilst under an inert atmosphere (e.g., argon) at ice-bath temperatures, preferably between 0° and 10° C.

An alternate method for the preparation of the α-keto ester is to subject the silyl ketene acetal to oxygen (in its in situ-prepared singlet state) by bubbling oxygen through the reaction mixture and irradiating the mixture with a 450 watt Hanovia lamp (Ace Glass) filtered through pyrex. The oxygen is sensitized in situ using standard techniques such as by contacting the silyl ketene acetal with hematoporphyrin.

The preparation of the desired compounds of formula I wherein X is sulfur (i.e. 4-amino-4,5-dihydro-2-thiophene carboxylic acid derivatives) is preferentially effected by a series of chemical reactions starting with derivatives of cysteine. In one such process, the amine moiety of 1-ethoxycarbonyl-2-mercapto-ethaniminium chloride is converted to its t-butoxycarbonyl derivative by reaction with di-t-butyl dicarbonate and the resulting product is treated with ethylbromoacetate, in situ, to form the desired N-[(1,1-dimethylethoxy)carbonyl]-S-(2-carboethoxyethyl) cysteine ethyl ester derivative. Of course, other N-protecting groups may similarly be employed. The so-prepared cysteine ethyl ester derivative is then subjected to a cyclization reaction using Dieckmann reaction conditions, preferably employing lithium diisopropylamine although others may similarly be utilized. The resulting β-ketoester (i.e., ethyl-4-[[(1,1-dimethylethoxy)-carbonyl]amino]tetrahydro-3-oxo-2-thiophene carboxylate) is sequentially reduced and dehydrated, preferably utilizing sodium borohydride, followed by mesyl chloride and the resulting 4-N-t-butoxycarbonyl-protected-amino-4,5-dihydro-2-thiophene carboxylic acid ester is deprotected according to standard conditions, such as lithium hydroxide hydrolysis, followed by reaction with dry hydrochloric acid gas.

Alternatively, the t-butoxycarbonyl protected cysteine starting material can be silylated (by reaction with chlorotrimethylsilane and bis-(trimethylsilyl)acetamide and the resulting product, when cyclized by reaction with freshly prepared lithium tetramethylpiperidine, will form a tetrahydro-2-thiophenecarboxylate bearing a silyl ether and alkyl ether at the 3-position thereof. The silyl ether is then removed by treatment with HF in the presence of tetrabutylammonium fluoride, and the product subjected to chemical reduction, preferably with sodium borohydride, to form the crude 3-hydroxy-N-t-BOC-protected-tetrahydro-2-carboxylate ester. This product may then be subjected to the same dehydration/deprotection reactions previously described to obtain the desired product.

The foregoing generally described processes are further illustrated by the following examples.

## EXAMPLE I

### (4S)4-Amino-4,5-dihydro-2-furancarboxylic acid

#### Step A: 2-[[(1,1-dimethylethyl)dimethylsilyl]oxy]methyl tetrahydro-3,4-furandiol

Tetrahydro-2-(hydroxymethyl)-3,4-furandiol (10.2 g, 76 mmole), (prepared from L-arabinose as described for the D-isomer by Rabinsohn and Fletcher, J.O.C., 32, 3452, 1968) in THF (100 ml) containing triethylamine (8 g, 80 mmole, 11.4 ml) and dimethylaminopyridine (100 mg) is treated dropwise with t-butyl dimethylsilyl chloride (12.0 g, 80 mmole) in THF (20 ml). The mixture is stirred at room temperature for 16 hours, then diluted with ether, filtered and concentrated. The product (12.2 g) is isolated by flash chromatography on silica gel using ethyl acetate-hexane (3:1) as eluant.

#### Step B: 2-[[[(1,1-dimethylethyl)dimethylsilyl]oxy]methyl]tetrahydro-3,4-furandiol bis(methylsulfonate)

To the diol (12.2 g, 49.1 mmole) in $CH_2Cl_2$ (200 ml) containing triethylamine (12.0 g, 120 mmole, 17,ml) at 0° is added mesyl chloride (12.5 g, 8.5 ml) and the mixture stirred at room temperature overnight. The mixture is then washed with water, $Na_2CO_3$ solution, dried and concentrated. The bismesylate (16.0 g)

is isolated by flash chromatography on silica gel using 30% ethyl acetate-hexane as eluant.

Step C: 4-Azido-2-[[[(1,1-dimethylethyl)dimethylsilyl]oxy]methyl]tetrahydro-3-furanol methylsulfonate

To the bismesylate (16.0 g, 49 mmole) in DMF (70 ml) is added $NaN_3$ (6.2 g, 55 mmole) and the mixture heated at 93° C for 20 hours. The mixture, on cooling, is poured into water and extraced with ether. The azide (5.6 g) is isolated by flash chromatography using 25% ethyl acetate as eluant.

Step D: Methyl 4-azidotetrahydro-3-[(methylsulfonyl)oxy]-2-furancarboxylate

The silyl ether (5.6 g, 16 mmole) in THF (50 ml) is treated with $(nBu)_4NF$ (18 ml of a 1 M solution) for 2 hours at room temperature. The solution is then diluted with $CH_2Cl_2$ and washed with brine. Flash chromatography on silica gel using ethyl acetate as eluant afforded the alcohol (3.3 g). This is dissolved in acetone (15 ml) and treated with an excess of Jones reagent for 16 hours. The acetone is evaporated off at room temperature, the residue treated with brine (15 ml) and extracted well with $CH_2Cl_2$. The organic phase is dried and concentrated, then treated with an ethereal solution of diazomethane. Flash chromatography using ethyl acetate as eluant afforded the ester (1.6 g).

Step E: Methyl 4-azido-4,5-dihydro-2-furancarboxylate

To the ester (1.6 g) in $CH_2Cl_2$ (20 ml) is added triethyl amine (2 ml) and the solution is stirred for 24 hours at room temperature, then concentrated. The product (600 mg) is isolated by flash chromatography using 20% ethyl acetate-hexane as eluant.

Step F: 4-Amino-4,5-dihydro-2-furancarboxylic acid

The azide (500 mg, 2.96 mmole) in methanol (10 ml) is treated with triethylamine (400 mg) and propanedithiol (432 mg, 4 mmole) for 48 hours. The mixture is then filtered and concentrated. The residue is treated with methanol (5 ml) and IN LiOH (5 ml) for 24 hours at room temperature. It is then diluted with water (15 ml) and extracted with $CH_2Cl_2$. The aqueous phase is acidified and extracted with $CH_2Cl_2$. The aqueous phase is then concentrated under reduced pressure. The residue is diluted with water, neutralized by the addition of 1 N $NH_4OH$ and applied to a Dowex AG 50W-X8 resin in the acid form. The column is eluted with water, then with 1 N $NH_4OH$. Concentration of the $NH_4OH$ eluate affords the amino acid (110 mg) which is recrystallized from acetone/water to give 86 mg.

EXAMPLE II

4-Amino-4,5-dihydro-2-furancarboxylic acid

Step A: 2,5-Dihydro-2-furancarboxylic acid

Into a three-neck one liter flask, fitted with a dry, ice-acetone condenser and cooled in a dry ice-acetone bath, was condensed 750 ml of ammonia. 2.5 g of sodium metal was then added and the resultant blue-black solution stirred for 15 minutes before distilling off 500 ml of ammonia into a similarly fitted second flask. 2-Furoic acid (15 g, 0.133 mole), dissolved in 90 ml of absolute ethanol, was then added by syringe to the stirred, distilled ammonia and the cooling bath removed. Lithium wire (2.8 g, 0.40 mole) was added in small pieces. The resultant blue color quickly faded as the ammonia reached reflux. The ammonia was then removed by distillation and the reaction residue placed under high vacuum for several minutes. After the last traces of ammonia had been removed, 100 ml of water was added to the residue and the pH adjusted to 8 using 6M HCl. Methylene chloride (300 ml) was then added followed by enough 6M HCl to bring the pH of the aqueous phase to 2. The organic phase was separated and the aqueous phase extracted once more with methylene chloride. Ether was then added, the aqueous phase saturated using solid sodium chloride and the layers separated. The combined organic extracts were then dried over magnesium sulfate, filtered and concentrated at ambient temperature to afford an oil (10.6 g).

Step B: Methyl-2,5-dihydro-2-furancarboxylate

Dicyclohexylcarbodiimide (19.2 g, 92.9 mmole) was suspended in 250 ml of methylene chloride and 4.2

ml of methanol syringed in. The stirred mixture was then chilled in an ice bath and the acid (10.6 g, 92.9 mmole) added as a solution in 50 ml of methylene chloride. Dimethylaminopyridine (1.1 g, 9.3 mmole) was added and the cooling bath removed. After stirring at room temperature overnight, the reaction mixture was filtered. The filtrate was washed with water, 0.25M HCl and then brine. The organic layer was dried over magnesium sulfate, filtered and concentrated to a small residue which was distilled at 30 mm Hg. The fraction boiling at 90-104 °C was collected to yield a water white liquid (3.5 g).

Step C: Methyl-tetrahydro-3-iodo-4-isocyanato-2-furancarboxylate

The ester (3.8 g, 30 mmole) was dissolved in 50 ml of methylene chloride and chilled in an ice bath. Freshly prepared silver isocyanate (4.9 g, 33 mmole) was added to the stirred solution followed by iodine (7.6 g, 30 mmole). The reaction mixture was stirred at 0 °C for 1 hour and then at room temperature for 3 hours. The reaction mixture was then filtered through Celite®, the Celite® pad washed with ether and the filtrate concentrated to a purple, oily solid (10 g) which was used without further purification in the next step.

Step D: Methyl-tetrahydro-3-iodo-4-[[[(4-methoxyphenyl)methoxy]carbonyl]amino]-2-furancarboxylate

To the crude iodide (10 g) was added 3:8 ml of p-methoxybenzyl alcohol and the mixture stirred at room temperature overnight. Chromatography, using 25% ethyl acetate-hexane, gave the desired product as a mixture of isomers (5.96 g).

Step E: Methyl-4,5-dihydro-3-iodo-4-[[[(4-methoxyphenyl)methoxy]carbonyl]amino]-2-furancarboxylate

To the iodo compound (5.96 g), in 50 ml of acetone, was added 1.7 g of diazobicyclooctane. The mixture was stirred at room temperature in the dark, overnignt and then filtered. The filtrate was diluted with ether and water, the layers separated and the organic phase washed with water, 0.1 M HCl and then brine. The organic layer was dried over magnesium sulfate, filtered and concentrated to give a white oily solid. Chromatography, using 35% ethyl acetate-hexane, afforded the desired compound as a white solid (1.97 g).

Step F: 4,5-Dihydro-4-[[[(4-methoxyphenyl)methoxy]carbonyl] amino]-2-furancarboxylic acid

The carbamate (500 mg, 1.6 mmole) was dissolved in 5 ml of tetrahydrofuran and 8 ml of methanol and chilled in an ice bath. Lithium hydroxide (3.6 ml of a 1M aqueous solution) was then added dropwise to the stirred solution. The reaction mixture was stirred at 0 °C for two hours after this addition and then poured into 100 ml of ethyl acetate and 50 ml of a mixture composed of (8:1:1) brine:water:1M HCl. The layers were separated and the organic phase was washed once with brine before being dried over magnesium sulfate, filtered and concentrated to give a white solid (447 mg).

Step G: 4-amino-4,5-dihydro-2-furancarboxylic acid

The acid (447 mg) was suspended in 6 ml of anisole under argon and chilled in an ice bath. To the stirred suspension was added trifluoroacetic acid (10 ml) dropwise. After the addition was completed, the cooling bath was removed and the reaction mixture stirred at room temperature for 1 hour. The reaction mixture was concentrated at ambient temperature and the residue placed under high vacuum to give an oil material. Hexane was added to the oil residue, stirred for several minutes and then decanted. This process was repeated once more. 150 ml of ether was then added and the mixture stirred at room temperature overnight. The supernatant liquid was decanted from the white solid which resulted, additional ether added and the mixture filtered. A white solid, weighing 187 mg after drying in a vacuum desiccator, was obtained.

EXAMPLE III

(S)-4-Amino-4,5-dihydro-2-furancarboxylic acid

Step A: N-[(1,1-dimethylethoxy)carbonyl]-L-glutamic acid-5-methyl ester

L-glutamic acid-γ-methyl ester (32.2 g, 0.2 moles) was suspended in 250 ml of dioxane-water (1:1 by volume) and triethylamine (42.0 ml, 0.3 moles) was added in one portion. The resultant solution was cooled in an ice bath and di-t-butyldicarbonate (50.6 ml, 0.22 moles) added in portions at a rate to control frothing.

The cooling bath was removed after the addition was completed and the reaction mixture was stirred at room temperature overnight. The reaction mixture was concentrated to one-half its original volume on a rotovap and then diluted with 100 ml of water and 250 ml of ethyl acetate. This mixture was poured into a separatory funnel, the layers separated and the aqueous phase washed once more with 250 ml of ethyl acetate. 350 ml of ethyl acetate was then added to the separatory funnel and the aqueous phase acidified by the addition 150 ml of 1 M hydrochloric acid in 25 ml portions, shaking after each addition. The acidified aqueous layer was extracted with 2 x 100 ml of ethyl acetate. The combined organic extracts were washed once with 0.5 M hydrochloric acid and once with brine, dried over magnesium sulfate, filtered and concentrated to an oil on a rotovap. Further concentration on a vacuum pump gave the desired compound as an oil (45.1 g, 86%). Trituration with ether-hexane gave a white solid, m.p. 74-77° C (uncorrected). NMR ($CDCl_3$) $\delta$ 1.46 (s, 9H, -$C(CH_3)_3$), 1.84-2.60 (m, 4H, -$CH_2$-$CH_2$), 3.67 (s, 1H, -$OCH_3$), 4.08-4.50 (m, 1H, -CH-N), 5.10-5.47 (m, 1H, -NH), 11.00 (br s, 1H, -$CO_2H$). Anal. Calcd. for $C_{11}H_{19}NO_6$: C, 50.57; H, 7.33; N, 5.36. Found: C, 50.40; H, 7.69; N, 5.08.

Step B: Methyl 4-[[(1,1-dimethylethoxy)carbonyl]amino]-5-hydroxypentanoate

N-t-butoxycarbonyl-L-glutamic acid-γ-methyl ester (24.8 g, 94.9 mmoles) was dissolved in 400 ml of tetrahydrofuran under argon and cooled to -23° C in a carbon tetrachloride-dry ice bath. Triethylamine (13.2 ml, 94.9 mmoles) was added to the stirred solution and, after five minutes, isobutylchloroformate (12.3 ml, 94.9 mmoles) was syringed in. Vigorous stirring was maintained during the isobutylchloroformate addition and a white precipitate began to form approximately one-half way into the addition. The resultant suspension was stirred at -23° C for ten minutes and then stirring was ceased and the precipitate allowed to settle for five minutes. This suspension was then transferred, using a transfer needle and an argon stream, to a rapidly stirred solution of sodium borohydride (10.8 g, 0.28 moles) in 250 ml of water, precooled to 0-5° C in an ice bath. The sodium borohydride solution was contained in a three-neck, three liter flask equipped with an overhead stirrer and an internal thermometer. Addition to the sodium borohydride solution was at a rate such that the internal temperature was maintained at less than 15° C at all times. Frothing occurred and ice was added to the sodium borohydride solution to aid cooling. Forty minutes after the addition was completed, the reaction mixture was poured into a separatory funnel containing 500 ml of methylene chloride and 300 ml of water. The layers were separated and the alkaline aqueous phase was extracted with 2 x 250 ml of methylene chloride. The combined organic extracts were washed with 400 ml of a mixture composed of (6:1:1) brine:1 M hydrochloric acid:water, dried over magnesium sulfate, filtered and concentrated on a rotovap to an oil. Flash chromatography on a 6 x 15 cm column using first 30% ethyl acetate-hexane (1 liter) and then 70% ethyl acetate-hexane gave the desired compound as a water white oil (19.3 g, 82%). NMR ($CDCl_3$) $\delta$ 1.41 (s, 9H, -$C(CH_3)_3$), 1.55-2.08 (m, 2H, -$CH_2$-), 2.41 (t, 2H, J = 7.5 Hz, -$CH_2CO_2$), 3.48-3.79 (m, 3H, -CH-N and -$CH_2$-O), 3.63 (s, 3H, -$OCH_3$), 5.06-5.33 (m, 1H, -NH). Anal. Calcd. for $C_{11}H_{21}NO_5$: C, 53.43; H, 8.56; N, 5.66. Found: C, 53.51; H, 8.49; N, 5.49.

Step C: Methyl 4-[[(1,1-dimethylethoxy)carbonyl]amino]-5-[[(dimethylethyl)dimethylsilyl]oxy]pentanoate

The alcohol (19.25 g, 77.8 mmoles) was dissolved in 200 ml of methylene chloride and cooled in an ice bath. t-Butyldimethylsilyl chloride (11.38 g, 75.5 mmoles), 4-dimethylaminopyridine (670 mg, 5.5 mmoles) and triethylamine (14.1 ml, 0.10 moles) were then added, in that order, with stirring. The cooling bath was removed and the reaction mixture stirred at room temperature overnight. The reaction mixture was poured into 200 ml of methylene chloride and 150 ml of water contained in a separatory funnel. The layers were separated and the organic phase washed with 2 x 150 ml of 0.5 M hydrochloric acid and once with brine. The organic layer was then dried over magnesium sulfate, filtered and concentrated on a rotovap to a brown oil. Flash chromatography on a 7 x 15 cm column using 12% ethyl acetate-hexane gave the desired compound as a water white oil (21.8 g, 80%). NMR ($CDCl_3$) $\delta$ 0.03 (s, 6H, -$Si(CH_3)_2$), 0.89 (s, 9H, -SiC-$(CH_3)_3$), 1.42 (s, 9H, -$OC(CH_3)_3$), 1.67-2.00 (m, 2H, -$CH_2$-), 2.39 (t, 2H, J = 7.5 Hz, -$CH_2CO_2$), 3.48-3.77 (m, 3H, -CH-N and -$CH_2$-O), 3.63 (s, 3H, -$OCH_3$), 4.42-4.78 (m, 1H, -NH). Anal. Calcd for $C_{17}H_{35}NSiO_5$: C, 56.47; H, 9.76; N, 3.87. Found: C, 56.52; H, 9.61; N, 4.14.

Step D: 1,1-Dimethylethyl-[1-[[(1,1-dimethylethyl)silyl]oxy)methyl]-4-methoxy-4-[(trimethylsilyl)oxy]-3-butenyl]carbamate

Diisopropylamine (19.44 ml, 0.14 moles) was dissolved in 150 ml of tetrahydrofuran and cooled to -78° C under argon. n-Butyl lithium (74.5 ml of a 1.78 M solution in hexane, 0.13 moles) was syringed in

and the solution stirred for fifteen minutes. The ester (21.8 g, 60.29 mmoles), dissolved in 30 ml of tetrahydrofuran and precooled to -78 °C, was then added over an eight to ten minute time span using a transfer needle and an argon stream. The resultant solution was stirred for five minutes and then trimethylsilyl chloride (23.0 ml, 0.18 moles) was added by syringe over three to five minutes. The reaction mixture was allowed to warm slowly to room temperature over a one and one-half hour period. The white suspension was concentrated at ambient temperature on a rotovap, diluted with 200 ml of hexane and suction filtered. The filtered material was washed with a further 15 ml of hexane and the combined filtrate concentrated at ambient temperature on a rotovap, followed by a vacuum pump, to give the desired compound as a pale yellow, oily liquid (30.5 g, 100%).

Step E: Methyl 4-[[(1,1-dimethylethoxy)carbonyl]amino]-5-[[(1,1-dimethylethyl)dimethylsilyl]oxy]-2-hydroxypentanoate

The silyl ketene acetal (33.4 g, 66.4 mmoles) was dissolved in 400 ml of hexane, under argon, and cooled in an ice bath. The reaction flask was also fitted with an overhead stirrer to ensure vigorous agitation. 100% m-chloroperoxybenzoic acid was added in one portion to the vigorously stirred solution and the reaction mixture stirred at 0-5 °C for one hour. The reaction mixture was then suction filtered to remove a white solid, the solid washed with an additional 500 ml of cold hexane and the combined filtrate poured into a separatory funnel containing 500 ml of ether and 300 ml of saturated sodium bicarbonate. The layers were separated and the organic phase washed with 3 x 300 ml of a mixture composed of (1:1) saturated sodium carbonate: water followed by 300 ml of brine. The organic layer was dried over magnesium sulfate, filtered and concentrated on a rotovap. The residue was dissolved in 200 ml of methanol and 35 ml of water and stirred at room temperature for seven hours. The reaction mixture was then concentrated to one-half its original volume on a rotovap and poured into a separatory funnel containing 300 ml of methylene chloride and 100 ml of a mixture composed of (2:1) brine:water. The layers were separated and the organic phase extracted with an additional 50 ml of methylene chloride. The combined organic extracts were dried over magnesium sulfate, filtered and concentrated on a rotovap. Flash chromatography on a 7 x 15 cm column using hexane (1.5 l) followed by 5% ethyl acetate-hexane (1.5 l) and finally 25% ethyl acetate-hexane (2.5 l) afforded the desired compound as an oil (6.0 g, 24%) and allowed for the recovery of 5 grams (21%) of methyl 4-[[(1,1-dimethylethoxy)carbonyl]amino]-5-[[(1,1-dimethylethyl)dimethylsilyl]oxy]pentanoate which could be recycled. NMR ($CDCl_3$) δ 0.09 (s, 5H, $-Si(CH_3)_2$), 0.91 (s, 9H, $-Si-C(CH_3)_3$), 1.48 (s, 9H, $-OC(CH_3)_3$), 1.68-2.21 (m, 2H, $-CH_2-$), 3.43-4.10 (m, 4H, $-CH_2-O$ and -CH-N and -OH), 3.75 (s, 3H, $-OCH_3$), 4.10-4.42 (m, 1H, -CH-O), 4.58-5.16 (m, 1H, -NH). Anal. Calcd for $C_{17}H_{35}NSiO_6$: C, 54.08; H, 9.34; N, 3.71. Found: C, 54.19; H, 9.53; N, 3.41.

Step F: Methyl 4-[[(1,1-dimethylethoxy)carbonyl]amino]-5-[[(1,1-dimethylethyl)dimethylsilyl]oxy]-2-oxopentanoate

To a solution of the alcohol (5.8 g, 15.4 mmoles) in 150 ml of methylene chloride was added pyridinium dichromate (28.9 g, 76.8 mmoles) in portions. After the addition was completed, the suspension was stirred at room temperature for three days. An additional 2.9 g (7.7 mmoles) of pyridinium dichromate was added on the fourth and again on the fifth days. A final 2.9 g of pyridinium dichromate was added on the eighth day of stirring. After eleven days, the reaction mixture was diluted with 750 ml of ether while stirring vigorously. The reaction mixture was then suction filtered through celite® magnesium sulfate. The filter pad was washed with an additional 350 ml of ether and the combined filtrate concentrated on a rotovap to approximately 200 ml. This residue was washed with 2 x 50 ml of 0.5 M hydrochloric acid and once with 50 ml of brine. The organic phase was then dried over magnesium sulfate, filtered and concentrated to a brown oil. Flash chromatography on a 5 x 15 cm column using 15% ethyl acetate-hexane as the eluant gave the desired compound as a water white oil (4.44 g, 77%). NMR ($CDCl_3$) δ 0.06 (s, 6H, $-Si(CH_3)_2$), 0.89 (s, 9H, $-SiC(CH_3)_3$), 1.44 (s, 9H, $-OC(CH_3)_3$), 3.03 (d, 2H, J=6 Hz, $-CH_2CO-$), 3.68 (d, 2H, J=4 Hz, $-CH_2-O$), 3.85 (s, 3H, $-OCH_3$), 3.96-4.30 (m, 1H, -CH-N), 4.79-5.12 (m, 1H, -NH). Anal. Calcd for $C_{17}H_{33}NSiO_6$: C, 54.37; H, 8.86; N, 3.73. Found: C, 54.26; H, 9.13; N, 3.48.

Alternate synthesis for preparation of product of Step F:

A solution of the silyl ketene acetal (489 mg, 0.97 mmoles) of Step D and hematoporphyrin (2.5 mg) in 4:1 $CH_3CN:CHCl_3$ (5 ml) was chilled in an ice bath. Oxygen gas was bubbled through the solution at about 250 ml/min while irradiating with a 450 watt Hanovia lamp (Ace Glass) filtered through pyrex.

After 30 min, an aliquot was removed and quenched in saturated $NaHCO_3$ solution. TLC analysis showed only a trace of the starting ester.

The solution was purged with argon and treated with 0.75 ml $Et_3N$ (5.5 eq.) for 90 min at 0° C. The solution was diluted with ether, washed with 0.5 N HCl, saturated $NaHCO_3$ solution and brine, and dried over $MgSO_4$. Filtration and concentration of the filtrate produced an oil (305 mg) from which 195 mg (54%) of the α-ketoester was isolated by flash chromatography. This sample displayed the see spectral characteristics and TLC behavior as a sample of α-ketoester prepared by alternate methods.

Step G: Methyl 4-[[(1,1-dimethylethoxy)carbonyl]amino]tetrahydro-2-hydroxy-2-furancarboxylate

To a solution of the α-ketoester (3.76 g, 10.0 mmoles) in 35 ml of tetrahydrofuran was added 6.5 ml of 2% aqueous perchloric acid. The resultant solution was stirred at room temperature for five hours (TLC shows reaction to be complete) and then poured into a separatory funnel containing 200 ml of ethyl acetate and 50 ml of a mixture composed of (3:1:1) brine: water:saturated aqeuous sodium bicarbonate. The layers were separated and the aqueous phase extracted with 2 x 20 ml methylene chloride. The combined organic extracts were dried over magnesium sulfate, filtered and concentrated on a rotovap. Flash chromatography on a 5 x 12 cm column using 45% ethylacetate-hexane as the eluant gave the desired compound, a water white oil (2.18 g, 84%), as a mixture of epimers. NMR ($CDCl_3$) δ 1.42 (s, 9H, $-OC(CH_3)_3$), 1.88-2.78 (m, 2H, $-CH_2-C-CO_2$), 3.76 and 3.78 (s, 3H, $-OCH_3$), 3.82-4.51 (m, 3H, -CH-N and $-CH_2-O$), 4.40 and 4.68 (br s, 1H, -OH), 5.18-5.63 (m, 1H, -NH). Anal. Calcd for $C_{11}H_{19}NO_6$: C, 50.57; H, 7.33; N, 5.36. Found: C, 50.82; H, 7.47; N, 5.37.

Step H: Methyl 4-[[(1,1-dimethylethoxy)carbonyl]amino]-4,5-dihydro-2-furancarboxylate

To a solution of the alcohol (2.48 g, 9.45 mmoles) in 40 ml of methylene chloride, cooled in an ice bath under argon, was added pyridine (1.54 ml, 18.98 mmoles) and then thionyl chloride (0.69 ml, 9.49 mmoles). After fifteen minutes, the cooling bath was removed and the reaction mixture was stirred at room temperature for an additional five hours. The reaction mixture was then added to a separatory funnel containing 60 ml of methylene chloride and 35 ml of 0.5 M hydrochloric acid. The layers were separated and the organic phase washed with 25 ml of brine, dried over magnesium sulfate, filtered and concentrated on a rotovap to a white solid. Flash chromatography on a 5 x 15 cm column using 35% ethyl acetate-hexane as the eluant gave the desired compound as a white solid (1.80 g, 78%), m.p. 94-96° C (uncorrected). NMR ($CDCl_3$) δ 1.40 (s, 9H, $-OC(CH_3)_3$), 3.76 (s, 3H,$-OCH_3$), 4.23 (dd, 1H, J = 4.5 and 10.5 Hz, -CH-O), 4.50 (dd, 1H, J = 9.0 and 10.5 Hz, -CH-O), 4.80-5.27 (m, 2H, -NH and -CH-N), 5.90 (d, 1H, J = 3 Hz, -C = CH). Anal. Calcd for $C_{11}H_{17}NO_5$: C, 54.31; H, 7.04; N, 5.76. Found: C, 54.07; H, 7.24; N, 5.47.

Step I: 4-[[(1,1-dimethylethoxy)carbonyl]amino]-4,5-dihydro-2-furancarboxylic acid

To a solution of the ester (1.80 g, 7.40 mmoles) in 25 ml of tetrahydrofuran and 35 ml of methanol, cooled in an ice bath, was added 16.28 ml of 1 M aqueous lithium hydroxide with stirring. The reaction mixture was stirred at ice bath temperature for ninety minutes and then poured into a separatory funnel containing 400 ml of ethyl acetate and 150 ml of a mixture composed of (8:1:1) brine:2 M hydrochloric acid:water. The layers were separated and the organic phase dried over magnesium sulfate, filtered and concentrated on a rotovap to a white solid. The solid was redissolved in 125 ml of ethyl acetate and washed with 2 x 20 ml of 0.1 M hydrochloric acid followed by 25 ml of brine. The organic phase was dried over magnesium sulfate, filtered and concentrated on a rotovap to give the desired compound as a white solid (1.55 g, 91%), m.p. 117-120° C (decomposes; uncorrected). NMR ($CDCl_3$) δ 1.43 (s, 9H, $-OC(CH_3)_3$), 4.25 (dd, 1H, J = 4.5 and 10.5 Hz, -CH-O), 4.52 (t, 1H, J = 10.5 Hz, -CH-O), 4.70-5.28 (m, 2H, -NH and -CH-N), 5.98 (d, 1H, J = 3Hz, -C = CH), 8.86 (br s, 1H, $-CO_2H$). Anal. Calcd for $C_{10}H_{15}NO_5$: C, 52.40; H, 6.60; N, 6.11. Found: C, 52.24; H, 6.66; N, 5.92.

Step J: (S)-4-Amino-4,5-dihydro-2-furancarboxylic acid

To 4.5 ml of freshly distilled trifluoroacetic acid, under argon and cooled in an ice bath, was added the acid (774 mg, 3.38 mmoles) in one portion with stirring. Immediate gas evolution was noted and, after fifteen minutes, the solution was added dropwise to 125 ml of cold, anhydrous ether with rapid stirring. The resultant white precipitate was stirred for fifteen minutes at ice bath temperature and then filtered under argon. The collected white solid was washed three times with 10 ml of cold anhydrous ether and then dried under vacuum. Chromatography on a 2 x 10 cm bed of ion exchange resin (Biorad AG50W-X8; $H^+$ form; 50-100 mesh) using 2 M ammonium hydroxide as the eluant gave the desired compound as a white solid

(305 mg, 70%), m.p. 193-195 °C (decomposes; uncorrected). NMR ($CF_3CO_2D$) δ 4.73-5.17 (m, 3H, $-CH_2O$ and -CH-N), 6.34 (d, 1H, -CH=C, J=3 Hz). Anal. Calcd for $C_5H_7NO_3$: C, 46.51; H, 5.46; N, 10.85. Found: C, 46.30; H, 5.48; N, 10.55.

Similar to the 2-furan carboxylic acids, the 4-amino-4,5-dihydro-2-thiophene carboxylic acids of this invention also contain an asymetric carbon atom and thus the products exist in enantiomorphic forms. The preparation of the individually desired enantiomers is effected by utilizing the appropriate isomer of the starting material (e.g., the D- or L- form of the starting cysteine derivative wherein the L-isomer yields the (R) isomer and the D-isomer yields the (S) isomer). In the event discriminating procedures are not utilized, obtention of the desired isomer may be effected according to standard techniques, such as separation of the racemic mixture with optically active acids or by the use of prep columns such as Prep Percol® columns.

The following specific examples illustrate the preferred methods of synthesis although such examples are not restrictive of the methodology which can be utilized. Obvious equivalency functioning procedures may be utilized depending upon factors fully appreciated by those skilled in the art.

## EXAMPLE IV

### 4-Amino-4,5-dihydro-2-thiophenecarboxylic acid

#### Step A: N-[(1,1-Dimethylethoxy)carbonyl](2-carboethoxyethyl)-L-cysteine ethyl ester

Trimethylamine 120 ml (0.88 mole) is added dropwise over a 10 minute period to a stirring ice bath cooled solution containing 40.7 g (0.22 mole) of cysteine ethyl ester hydrochloride, 51.0 g (0.227 mole) of di-tert-butyldicarbonate, and 400 ml of dry $CH_2Cl_2$ under a positive argon atmosphere. After an additional 20 minutes the cooling bath is removed and the reaction mixture is stirred at 25 °C for 4 hours. The reaction mixture is then once again cooled with an ice bath and 31 ml (0.264 mole) of ethyl bromoacetate is added and allowed to react for 1 hour at 0 °C and 2 hours at 25 °C. After removing the bulk of the solvent at reduced pressure the mixture is transferred to a separatory funnel, diluted with 300 ml of ether, and extracted with 200 ml of water. The organic layer is washed successively with 50 ml water and 50 ml brine and dried over $MgSO_4$. Evaporation of the solvent at reduced pressure affords 75.2 g of a light yellow-orange oil suitable for use in Step B.

#### Step B: Ethyl 4-[[(1,1-dimethylethoxy)carbonyl]amino]tetrahydro-3-oxo-2-thiophenecarboxylate

To a solution of LDA [prepared by the addition of 9.8 ml of 1.75 M n-BuLi (0.017 mole) to a solution of 2.4 ml of diisopropylamine (0.017 mole) and 15 ml of THF at 0 °C] cooled to -70 °C (dry ice/acetone) under a positive stream of argon is added dropwise over a 5 minute period 2.83 g of the unpurified cysteine derivative in 60 ml of THF. After stirring for 1 hour at -70 °C the reaction mixture is allowed to warm slowly to 20 °C at which point it is poured into a separatory funnel containing 100 ml of ether and sufficient 1/2 saturated acidic (HCl) brine to adjust the aqueous layer to pH 3 after equilibration. The organic layer is washed with 1/2 saturated brine, brine and dried over $MgSO_4$. The solvent is removed at reduced pressure to afford 2.21 g of oil which affords 1.07 g (44% from cysteine ethyl ester) of nearly pure β-ketoester following flash chromatography (5 cm column) on silica gel eluting sequentially with 20, 30 and 40% EtOAc in hexane.

#### Step C: 4-[[(1,1-dimethylethoxy)carbonyl]amino]-4,5-dihydro-2-thiophenecarboxylic acid

To a stirring solution of 5.16 g (0.178 mole) of the racemic β-ketoester in 40 ml of absolute ethanol cooled to -5 ° to -10 °C (ice/methanol bath) is added 340 mg (0.009 mole) of $NaBH_4$. After 25 minutes 5 ml of acetone is added and stirring and cooling is continued for an additional 20 minutes. The reaction mixture is then shaken in a separatory funnel containing 200 ml of EtOAc and 50 ml of 1/2 saturated brine, washed with brine, and dried over $MgSO_4$. The solvent is removed under reduced pressure to afford 5.25 g of the crude alcohol.

To a stirring ice bath cooled solution of the above alcohol, 8.3 ml (0.06 mole) of $Et_3N$, and 60 ml of $CH_2Cl_2$ under positive argon pressure is added dropwise 2.1 ml (0.027 mole) of mesyl chloride. After stirring for 2.5 hours at 0 °C the reaction which was judged complete by TLC is poured into 200 ml of ether, washed successively with 3 x 30 ml water and brine, and dried over $MgSO_4$. The unsaturated ester so obtained after removal of the solvent at reduced pressure is dissolved in 60 ml ethanol. To the stirring ice

bath cooled solution is added 25 ml of 1N LiOH. After 1 hour at 0°C the reaction mixture is poured into a separatory funnel containing 150 ml of EtOAc, 70 ml of brine, and 25 ml of 1N HCl and extracted. The separated aqueous layer is re-extracted with 30 ml of fresh EtOAc, and the combined organic layer is washed with 70 ml of brine and dried over $MgSO_4$. The solvent is removed under reduced pressure and the residue purified by flash chromatography (6 cm column) eluting with 30, 38 and 45% EtOAc in hexane containing 1% acetic acid to afford 2.62 g (60% yield) of the crystalline acid, mp. 154-155°C. A recrystallized (ether/hexane) sample afforded correct combustion analysis. % Calcd: C, 48.96; H, 6.16; N, 5.17; S, 13.07%. % Found: C, 48.85; H, 6.14; N, 5.15; S, 12.99.

Step D: 4-amino-4,5-dihydro-2-thiophenecarboxylic acid

An ice bath cooled solution containing 2.60 g (10.6 mMole) of the t-Boc acid in 100 ml of EtOAc is saturated with dry HCl. The cooling bath is then removed and the reaction mixture is allowed to stand for 1 hour under a positive nitrogen atmosphere. The precipitated solid is collected, washed successively with EtOAc and ether to afford, after drying in vacuo at 25°C, 1.20 g of a tan powder composed of amino acid hydrochloride and ammonium chloride. The solid is dissolved in water and the pH adjusted to 8-9 with 2N $NH_4OH$. The solution is then placed on a BioRad AG 50W-X8 ion exchange column (HCl form). The purified amino acid is eluted with 2N $NH_4OH$ and the water removed at reduced pressure (30°C bath) to afford a tan solid. The solid is transferred to a frit, washed with EtOAc and ether, and dried at high vacuum in a drying pistol (acetone) to afford 556 mg (34% yield) of the analytically pure amino acid. Analysis % Calcd: C, 48.96; H, 6.16; N, 5.71; S, 13.07. % Found: C, 48.85; H, 6.14; N, 5.55; S, 12.99.

EXAMPLE V

(S)4-Amino-4,5-dihydro-2-thiophenecarboxylic acid

Step A: N-[(1,1-Dimethoxyethoxy)carbonyl]-N-(trimethylsilyl)-S-(2-carboethoxyethyl)-D-cysteine ethyl ester

Into a dry argon-flushed flask containing 12.5 g (37.3 mmoles) of N-[1,1-dimethylethoxy)carbonyl]-S-2-(carboethoxy ethyl)-D-cysteine ethyl ester is added 28 ml (112 mmoles) of bis(trimethylsilyl)acetamide and 0.5 ml (4 mmoles) of chlorotrimethylsilane. The mixture is heated for 7 hours in a 130°C oil bath and then bulb-to-bulb distilled under a 50 micron vacuum. The 70°-80°C distilled material is discarded and the 130°-140°C distilled material is collected to afford 14.0 g of the desired product.

Step B: Ethyl-4-[[(1,1-dimethylethoxy)carbonyl]amino]-3-ethoxy-3-[(trimethylsilyl)oxy]tetrahydro-2-thiophene carboxylate

A 37.5 mmole solution of lithium tetramethylpiperidide (prepared at ice-bath temperature by adding 23.6 ml of 1.58 M n-butyl Lithium to 7 ml (41 mmole) of 2,2,6,6-tetramethylpiperidine in 50 ml of THF) was added over a 10 minute period to a stirring solution containing 14.0 g (34.4 mmole) of the silylated substrate of Step A in 190 ml THF which was maintained under a positive argon atmosphere at -65°C. Following the addition, the reaction mixture was held for 30 minutes at -70°C and allowed to warm slowly (~1 hr.) to -35°C at which point the solution was cooled to -60°C and quenched with 3.5 ml of acetic acid. The reaction mixture was poured into a separatory funnel containing 300 ml of ether, washed successively with 150 ml water, sufficient 0.5 N HCl to afford an acidic aqueous phase, and brine, and dried over $MgSO_4$. Removal of the solvent under reduced pressure afforded 12.4 g of an oil which was purified by flash chromotography (eluting successively with 7.5, 10, 20% EtOAc/hexane) to afford 7.53 g of product as a colorless oil.

Step C: Ethyl 4-[[(1,1-dimethylethoxy)carbonyl]amino]tetrahydro-3-hydroxy-2-thiophenecarboxylate

Into a stirring 25°C solution containing 7.53 g (18.5 mmole) of the silyl ether of Step B and 93 ml of a 1M ethanolic HF solution (prepared by mixing 1 part 48% aqueous HF with 29 parts of absolute ethanol) was added 18.5 ml of a 1 M solution of tetrabutylammonium fluoride in THF. After stirring for 40 minutes the solution was cooled with an ice/methanol bath and 520 mg (13.6 mmole) of $NaBH_4$ was added portionwise at such a rate that the internal temperature did not rise above 0°C. Following the addition the reaction mixture was stirred for an additional 30 minutes at which point the remaining hydride reagent was destroyed by the addition of 10 ml of acetone. One ml of acetic acid was added after an additional 10

minutes and the reaction mixture was concentrated to a volume of 50 ml under reduced pressure. The concentrated mixture was poured into a separatory funnel containing 300 ml EtOAc and washed successively with 100 ml of 1/2 saturated brine, 2 x 30 ml water, and 50 ml brine, and dried over $MgSO_4$. Removal of the solvent at reduced pressure afforded 10.4 g of an oil which was used without purification.

Step D: Ethyl-4-[[(1,1-dimethylethoxy)carbonyl]amino]-4,5-dihydro-2-thiophenecarboxylate

Into an ice cooled stirring solution containing the crude alcohol obtained in Step C, 75 ml of $CH_2Cl_2$, and 10.5 ml (74 mmole) of $Et_3N$ was added 2.9 ml (37 mmole) of mesyl chloride while maintaining the system under a positive argon atmosphere. The reaction mixture was stirred an additional 5 minutes at 0 °C and then 3 hours at 25 °C. The reaction mixture was poured into a separatory funnel containing 200 ml $Et_2O$ and successively washed with 70 ml water, 1N HCl until the aqueous wash tested acidic, 15 ml water and 50 ml brine, and dried over $MgSO_4$. Removal of the solvent at reduced pressure afforded 6.5 g of a brown oil which was purified by flash chromatography (eluting with 15% and then 20% EtOAc/hexane) to yield 3.97 g of product. This material exhibited the following optical rotation

$$[\alpha]_D^{amb} = +122.8°$$

at a concentration of 0.5 M in ethanol. The highest rotation observed for the (R) enantiomer under similar conditions was -128.1 °.

Step E: 4-[[(1,1-dimethylethoxy)carbonyl]amino]-4,5-dihydro-2-thiophenecarboxylic acid

To a stirring ice cooled solution containing 3.7 g (13.5 mmole) of the ethyl ester in 45 ml of ethanol was added 19 ml of 1 N LiOH. After stirring for 1 hour the reaction mixture was extracted in a separatory funnel containing 100 ml EtOAc, 40 ml brine, 20 g ice, and 4 ml of 6N HCl, The organic phase was washed with 2 x 10 ml $H_2O$ and dried over $MgSO_4$. Removal of the solvent under reduced pressure afforded 3.2 g of a crystalline solid, 99% yield. This material was recrystallized from EtOAc/hexane to afford 1.2 g of fluffy white crystals. The acid before recrystallization was 99.1% (S) and 0.9% (R).

Step F: 4-amino-4,5-dihydro-2-thiophenecarboxylic acid

The recrystallized acid of step E (1.2 g) was dissolved in 50 ml EtOAc, cooled with an ice bath, and dry HCl gas was bubbled through the solution for 3 minutes. Ten minutes later the cooling bath was removed. After an additional 1 1/2 hours the reaction mixture was filtered, and the solids washed successively with EtOAc and ether to afford 610 mg of the amino acid hydrochloride as an off white solid.

Illustrative of the pharmaceutically acceptable salts of the compounds of this invention include nontoxic acid addition salts formed with inorganic acids, such as, hydrochloric, hydrobromic, sulfuric and phosphoric acid, and organic acids such as methanesulfonic, salicylic, maleic, malonic, tartaric, citric, and ascorbic acids; and non-toxic salts formed with inorganic or organic bases such as those of alkali metals, for example, sodium, potassium and lithium, alkaline earth metals, for example, calcium and magnesium, light metals of Group III A,for example, aluminum; organic amines such as primary, secondary or tertiary amines, for example, cyclohexylamine, ethylamine, pyridine, methylaminoethanol, ethanolamine and piperazine. The salts can be prepared by conventional means.

As stated above, the compounds of this invention possess the inherent characteristics of inhibiting $\gamma$-aminobutyric acid transaminase which results in an increase in brain levels of $\gamma$-aminobutyric acid. Thus, the compounds are useful in the treatment of disorders of the central nervous system, particularly those dealing with the function of involuntary movement generally associated with seisure disorders of epilepsy.

The ability of the compounds of this invention to inhibit $\gamma$-aminobutyric acid transaminase and raise brain GABA levels is determined in vitro by the methods of Lippert et al. (Eur. J. Biochem. 71:441, 1977) and in vivo by Jung et al. (J. Neurochem. 28:717, 1977). $\gamma$-Aminobutyric acid levels are markedly increased in mouse brains after treatment with compounds of this invention at doses ranging from 0.5 mg/kg to 10 mg/kg of body weight by parenteral or oral routes. This ability is further demonstrated by the protective effect (anti-epileptic), at doses ranging from 0.5 mg/kg to 25 mg/kg, against convulsions elicited by an intravenous dose of 3-mercaptopropionic acid (100 mg) according to the general method described by Sarhan and Seilar, J. Neuroscience Res., 4 (1979) 399-421 which is used to evidence anti-epileptic activity. Therefore, based on the foregoing results, as well as by comparison with other known compounds useful in

the treatment of epilepsy the dose range of the compounds of this invention for the treatment of epilepsy is 0.1 mg -25 mg per kilogram of body weight per day, depending upon the age of the patient, severity of the disease state and other factors as determined by the attending diagnostician.

In addition to their use in the treatment of epilepsy, in their effect on the central nervous system the compounds of the invention may also be used in the treatment of schizophrenia, tardive dyskinesia, muscle spasms and, the compounds also exhibit analgesic effects. Standard laboratory methodology, in conjunction with compounds with known chemotherapeutic agents useful for the foregoing, may readily be utilized to determine the optimal doses for each of these indications.

The compounds of this invention can be administered orally or parenterally to animals, particularly warm blooded animals and mammals and humans either alone or in the form of pharmaceutical preparations containing as the active ingredient compounds of this invention to achieve the desired effect. Pharmaceutical preparations containing compounds of this invention and conventional pharmaceutical carriers can be employed in unit dosage forms such as solids, for example, tablets, pills and capsules or liquid solutions, suspensions or elixirs for oral administration or liquid solutions, suspensions and emulsions for parenteral use. The quantity of compounds administered can vary over a wide range to provide from about 0.1 mg/kg to about 300 mg/kg of body weight of the patient per day. Unit doses of these compounds can contain, for example, from about 50 mg to 2000 mg of the compounds and may be administered, for example, from 1 to 4 times daily. Following are illustrative examples of pharmaceutical preparations containing the compounds of this invention:

| | Per Tablet |
|---|---|
| (a) 4-amino-4,5-dihydro-2-furancarboxylic acid | 100.0 mg |
| (b) wheat starch | 15.0 mg |
| (c) lactose | 33.5 mg |
| (d) magnesium stearate | 1.5 mg |

A portion of the wheat starch is used to make a granulated starch paste which together with the remainder of the wheat starch and the lactose is granulated, screened and mixed with the active ingredient (a) and the magnesium stearate. The mixture is compressed into tablets weighing 150 mg each.

An illustrative composition for a parenteral injection is the following wherein the quantities are on a weight to volume basis:

| | Amount |
|---|---|
| (a) 4-amino-4,5-dihydro-2-furancarboxylic acid | 100.0 mg |
| (b) sodium chloride | q.s |
| (c) water for injection to make | 20 ml |

The composition is prepared by dissolving the active ingredient (a) and sufficient sodium chloride in water for injection to render the solution isotonic. The composition may be dispensed in a single ampule containing 100 mg of the active ingredient for multiple dosage or in 20 ampules for single dosage.

An illustrative composition for hard gelatin capsules is as follows:

| | Amount |
|---|---|
| (a) 4-amino-4,5-dihydro-2-furancarboxylic acid | 200.0 mg |
| (b) talc | 35.0 mg |

The composition is prepared by passing the dry powders of (a) and (b) through a fine mesh screen and mixing them well. The powder is then filled into No. 0 hard gelatin capsules at a net fill of 235 mg per capsule.

**Claims**

**Claims for the following Contracting States : BE, CH, DE, FR, GB, IT, LU, NL, SE**

**1.** A compound of the formula

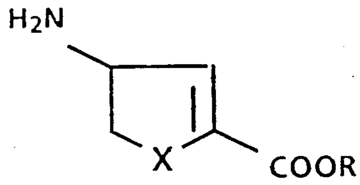

including the individual enantiomers and racemic mixtures thereof and the pharmaceutically acceptable salts thereof, wherein R is hydrogen or ($C_1$-$C_6$)alkyl, and X is oxygen or sulfur.

**2.** A compound of Claim 1 wherein X is oxygen.

**3.** A compound of Claim 1 wherein X is sulfur.

**4.** A compound of claims 2 and 3 wherein R is hydrogen.

**5.** The (S) enantiomer of a compound of claim 2, said compound being (S) 4-amino-4,5-dihydro-2-furancarboxylic acid.

**6.** A compound of claim 3 wherein R is hydrogen, said compound being 4-amino-4,5-dihydro-2-thiophenecarboxylic acid.

**7.** A process for the preparation of 4-amino-4,5-dihydro-2-furancarboxylic acid which comprises reacting 4-[[(1,1-dimethylethoxy)carbonyl]amino]-4,5-dihydro-2-furancarboxylic acid with trifluoroacetic acid, under an inert atmosphere.

**8.** A process of claim 7 wherein the inert gas is argon and the reaction is conducted at temperatures in the range of about 0° to 10° C.

**9.** A process of claim 7 wherein the trifluoroacetic acid is neat.

**10.** In the process for producing 4-amino-4,5-dihydro-2-furancarboxylic acid, and the lower alkyl esters thereof according to claim 7, the step which comprises reacting methyl 4-[[(1,1-dimethylethyl)-dimethylsilyl]oxy]-2-oxopentanoate, and equivalents thereof with a mineral acid to form methyl 4-[[(1,1-dimethylethoxy)carbonyl]amino]tetrahydro-2-hydroxy-2-furancarboxylate, said reaction being effected at about room temperature.

**11.** A process according to claim 10 wherein the resulting product is reacted with a dehydrating reagent to produce methyl 4-[[(1,1-dimethylethoxy)carbonyl]amino]-4,5-dihydro-2-furancarboxylate.

**12.** A process according to claim 11 wherein the dehydrating agent is thionylchloride and the reaction is effected in pyridine.

**13.** A process for preparing a 4-amino-4,5-dihydro-2-thiophenecarboxylic acid and the $C_{1-6}$ alkyl esters thereof which comprises deprotecting an N-protected-4-amino-4,5-dihydro-2-thiophenecarboxylic acid and optionally esterifying said acid by standard deprotecting and esterifying procedures.

**14.** A process for preparing a 4-amino-4,5-dihydro-2-thiophenecarboxylic acid and the $C_{1-6}$ alkyl esters thereof which comprises chemically reducing and dehydrating a 3-hydroxy or 3-oxo derivative of an N-protected-4-amino-4,5-dihydro-2-thiophenecarboxylic acid, or ester thereof, and optionally esterifying or deesterifying the resulting products by standard techniques.

**15.** A process according to claim 14 wherein the chemical reduction agent is sodium borohydride and the dehydrating agent is mesylchloride.

**16.** A process according to claims 13 and 14 wherein the N-protecting group is t-butoxycarbonyl.

**Claims for the following Contracting State : AT**

1. A process for preparing a compound of the formula I

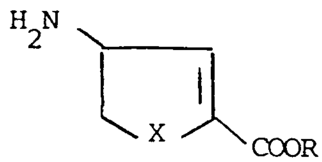

I

including the individual enantiomers and racemic mixtures thereof and the pharmaceutically acceptable salts thereof, wherein R is hydrogen or ($C_1$-$C_6$)lower alkyl, and X is oxygen or sulfur, which comprises:

a) when a compound of formula I wherein X is oxygen is desired, deprotecting an N-protected-4-amino-4,5-dihydro-2-furancarboxylic acid or ($C_1$-$C_6$)alkyl ester thereof by reaction under acidic conditions,

b) when a compound of formula I wherein X is sulfur is desired, deprotecting an N-protected-4-amino-4,5-dihydro-2-thiophenecarboxylic acid or ($C_1$-$C_6$)lower alkyl ester thereof by reaction under acidic conditions,

c) when a compound of formula I wherein R is hydrogen is obtained according to step a) or b), optionally converting it into the corresponding ($C_1$-$C_6$)alkyl ester by esterification procedures; or, when a lower alkyl ester is obtained according to step a) or b), optionally converting it into another ($C_1$-$C_6$)lower alkyl ester by transesterification procedures.

2. A process according to claim 1 for preparing a compound of formula I wherein R is hydrogen.

3. A process for preparing a compound of claim 1 wherein X is oxygen characterized in that a ($C_1$-$C_6$)-alkyl-4-azido-4,5-dihydro-2-furancaboxylate is reduced to give the corresponding ($C_1$-$C_6$)alkyl-4-amino-4,5-dihydro-2-furancarboxylate.

4. A process according to claim 1 for preparing a compound of formula I wherein X is oxygen in a racemic form, which comprises

a) converting a ($C_1$-$C_6$)alkyl tetrahydro-3-iodo-4-isocyanato-2-furancarboxylate into the corresponding carbamate by reaction with an alcohol,

b) transforming the obtained ($C_1$-$C_6$)alkyl-tetrahydro--3-iodo-4-carbamate-2-furancarboxylate into the corresponding ($C_1$-$C_6$)alkyl-4,5-dihydro-4-carbamate-2-furancarboxylate by reacting it with diazabicyclooctane in acetone,

c) hydrolyzing the alkyl ester bond to give the corresponding free acid, and

d) submitting the thus obtained 4,5-dihydro-4-carbamate-2-furancarboxylic acid to acid hydrolysis to give the corresponding amine derivative of formula I.

5. A process according to Claim 1 for preparing a compound wherein X is oxygen characterized in that the deprotection of an N-protected-4-amino-4,5-dihydro-2-furancarboxylic acid is obtained by means of trifluoroacetic acid, under inert atmosphere.

6. A process according to Claim 5 wherein the inert gas is argon and the reaction is conducted at temperatures between 0 °C and 10 °C.

7. A process according to claim 5 wherein the trifluoroacetic acid is neat.

8. In a process according to claim 1 for preparing a compound of formula I wherein X is oxygen, the step which comprises reacting a ($C_1$-$C_6$)alkyl-4-(N-protected)amino-5-[[(1,1-dimethylethyl)dimethylsilyl]-oxy]-2-oxopentanoate with a mineral acid to form a ($C_1$-$C_6$)alkyl-4-(N-protected)-amino-tetrahydro-2-hydroxy-2-furancarboxylate, said reaction being effected at room temperature.

9. In a process according to claim 1 for preparing a compound of formula I wherein X is oxygen, the step which comprises reacting a ($C_1$-$C_6$)alkyl-4-(N-protected)-amino-tetrahydro-2-hydroxy-2-furancarboxylate with a dehydrating agent to give a ($C_1$-$C_6$)alkyl-4-(N-protected)-amino-4,5-dihydro-2-furancarboxylate.

**10.** A process according to claim 9 wherein the dehydrating agent is thionyl chloride and the reaction is conducted in the presence of pyridine.

**11.** A process according to Claims 4, 5, 8 or 9 wherein the ($C_1$-$C_6$)alkyl group is a methyl group.

**12.** In a process according to Claim 1 for preparing a compound of formula I wherein X represents sulfur, the step which comprises dehydrating a ($C_1$-$C_6$)alkyl-4-(N-protected)amino-tetrahydro-3-hydroxy-2-thiophene carboxylate.

**13.** A process according to claim 12 wherein the dehydratation is conducted in the presence of mesyl chloride.

**14.** In a process according to claims 1 and 12 for preparing a compound of formula I wherein R represents sulfur, the step which comprises preparing a ($C_1$-$C_6$)alkyl-4-(N-protected)amino-tetrahydro-3-hydroxy-2-thiophene carboxylate by reducing a ($C_1$-$C_6$)alkyl-4-(N-protected)amino-tetrahydro-3-oxo-2-thiophene carboxylate in the presence of sodium borohydride.

**15.** A process according to claim 14 wherein the ($C_1$-$C_6$)alkyl-4-(N-protected)amino-tetrahydro-3-hydroxy-2-thiophene is obtained by treating a ($C_1$-$C_6$)alkyl-4-(N-protected)-amino-3-alkoxy-3-silyloxy-tetrahydro-2-thiophene carboxylate with HF in the presence of tetrabutylammonium fluoride.

**16.** A process according to Claims 1, 4, 5, 8, 9, 12, 14 or 15 wherein the N-protecting group is t-butoxycarbonyl.

**17.** A process according to Claims 12, 14 or 15 wherein the ($C_1$-$C_6$)alkyl group is an ethyl group.

**Revendications**
**Revendications pour les Etats contractants suivants : BE, CH, DE, FR, GB, IT, LU, NL, SE**

**1.** Composé de formule

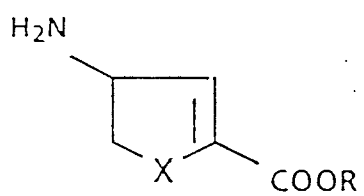

y compris les énantiomères individuels et leurs mélanges racémiques, et leurs sels pharmaceutiquement acceptables, où R est un hydrogène ou un alkyle en $C_1$-$C_6$ et X est un oxygène ou un soufre.

**2.** Composé selon la revendication 1, où X est un oxygène.

**3.** Composé selon la revendication 1, où X est un soufre.

**4.** Composé selon la revendication 2 ou 3, où R est un hydrogène.

**5.** L'énantiomère (S) d'un composé selon la revendication 2, ledit composé étant l'acide (S)-4-amino-4,5-dihydro-2-furannecarboxylique.

**6.** Compose selon la revendication 3, où R est un hydrogène, ledit composé étant l'acide 4-amino-4,5-dihydro-2-thiophènecarboxylique.

**7.** Procédé pour la préparation d'acide 4-amino-4,5-dihydro-2-furannecarboxylique, qui comprend la réaction de l'acide 4-[[(1,1-diméthyléthoxy)carbonyl]amino]-4,5-dihydro-2-furannecarboxylique avec l'acide trifluoroacétique sous une atmosphère inerte.

**8.** Procédé selon la revendication 7, dans lequel le gaz inerte est l'argon et la réaction est effectuée à une température dans la gamme d'environ 0° à 10° C.

**9.** Procédé selon la revendication 7, dans lequel l'acide trifluoroacétique est sans solvant.

**10.** Dans le procédé de production de l'acide 4-amino-4,5-dihydro-2-furannecarboxylique et de ses esters alkyliques inférieurs selon la revendication 7, l'étape qui comprend la réaction du 4-[[(1,1-diméthyléthyl)diméthylsilyl]oxy]-2-oxopentanoate de méthyle et ses équivalents avec un acide minéral pour former le 4-[[(1,1-diméthyléthoxy)carbonyl]amino]tétrahydro-2-hydroxy-2-furanne carboxylate de méthyle, ladite réaction étant effectuée au voisinage de la température ordinaire.

**11.** Procédé selon la revendication 10, dans lequel le produit obtenu est mis à réagir avec un agent déshydratant pour produire le [[(1,1-diméthyléthoxy)carbonyl]amino]-4,5-dihydro-2-furannecarboxylate de méthyle.

**12.** Procédé selon la revendication 11, dans lequel l'agent déshydratant est le chlorure de thionyle et la réaction est effectuée dans la pyridine.

**13.** Procédé pour préparer un acide 4-amino-4,5-dihydro-2-thiophènecarboxylique et ses esters d'alkyle en $C_1$-$C_6$, qui comprend la déprotection d'un acide 4-amino-4,5-dihydro-2-thiophènecarboxylique N-protégé et facultativement l'estérification dudit acide selon des modes standard de déprotection et d'estérification.

**14.** Procédé pour préparer un acide 4-amino-4,5-dihydro-2-thiophènecarboxylique et ses esters d' alkyle en $C_1$-$C_6$, qui comprend la réduction et la déshydratation chimiques d'un dérivé 3-hydroxy ou 3-oxo d'un acide 4-amino-4,5-dihydro-2-thiophènecarboxylique N-protégé ou d'un ester de celui-ci et facultativement l'estérification ou la désestérification des produits obtenus selon des techniques standard.

**15.** Procédé selon la revendication 14, dans lequel l'agent de réduction chimique est le borohydrure de sodium et l'agent déshydratant est le chlorure de mésyle.

**16.** Procédé selon les revendications 13 et 14, dans lequel le groupe N-protecteur est un groupe tert-butoxycarbonyle.

**Revendications pour l'Etat contractant suivant : AT**

**1.** Procédé pour préparer un composé de formule I

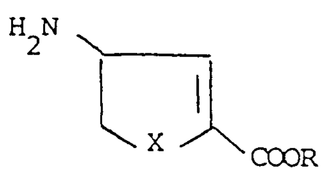

I

y compris les énantiomères individuels et leurs mélanges racémiques, et leurs sels pharmaceutiquement acceptables, dans laquelle R est un hydrogène ou un alkyle inférieur en $C_1$-$C_6$ et X est un oxygène ou un soufre, qui comprend :

a) lorsqu'on désire un composé de formule I dans laquelle X est un oxygène, la déprotection d'un acide 4-amino-4,5-dihydro-2-furannecarboxylique N-protégé ou d'un ester d'alkyle en $C_1$-$C_6$ de celui-ci par réaction dans des conditons acides,

b) lorsqu'on désire un composé de formule I dans laquelle X est un soufre, la déprotection d'un acide 4-amino-4,5-dihydro-2-thiophènecarboxylique N-protégé ou d'un ester alkylique inférieur en $C_1$-$C_6$ de celui-ci par réaction dans des conditions acides, et

c) lorsqu'on obtient un composé de formule I dans laquelle R est un hydrogène selon l'étape a) ou b), facultativement sa conversion en l'ester d'alkyle en $C_1$-$C_6$ correspondant selon des opérations d'estérification ; ou, lorsqu'on obtient un ester d'alkyle inférieur selon l'étape a) ou b), facultativement sa conversion en un autre ester d'alkyle inférieur en $C_1$-$C_6$ selon des opérations de

transestérification.

2. Procédé selon la revendication 1 pour préparer un composé de formule I dans laquelle R est un hydrogène.

3. Procédé pour préparer un composé selon la revendication 1 dans lequel X est un oxygène, caractérisé en ce que l'on réduit un 4-azido-4,5-dihydro-2-furannecarboxylate d'alkyle en $C_1$-$C_6$ pour obtenir le 4-amino-4,5-dihydro-2-furannecarboxylate d'alkyle en $C_1$-$C_6$ correspondant.

4. Procédé selon la revendication 1 pour préparer un composé de formule I dans laquelle X est un oxygène, sous une forme racémique, qui comprend
   a) la conversion d'un tétrahydro-3-iodo-4-isocyanato-2-furannecarboxylate d'alkyle en $C_1$-$C_6$ en le carbamate correspondant par réaction avec un alcool,
   b) la transformation du tétrahydro-3-iodo-4-carbamate-2-furannecarboxylate d'alkyle en $C_1$-$C_6$ obtenu en le 4,5-dihydro-4-carbamate-2-furannecarboxylate d'alkyle en $C_1$-$C_6$ correspondant par réaction avec le diazabicyclottane dans l'acétone,
   c) l'hydrolyse de la liaison ester alkylique pour former l'acide libre correspondant, et
   d) l'hydrolyse acide de l'acide 4,5-dihydro-4-carbamate-2-furannecarboxylique ainsi obtenu pour former le dérivé de type amine correspondant de formule I.

5. Procédé selon la revendication 1 pour préparer un composé dans lequel X est un oxygène, caractérisé en ce que l'on effectue la déprotection d'un acide 4-amino-4,5-dihydro-2-furannecarboxylique N-protégé par l'acide trifluoroacétique sous une atmosphère inerte.

6. Procédé selon la revendication 5, dans lequel le gaz inerte est l'argon et la réaction est effectuée à une température entre $0^\circ$ C et $10^\circ$ C.

7. Procédé selon la revendication 5, dans lequel l'acide trifluoroacétique est sans solvant.

8. Dans un procédé selon la revendication 1 pour préparer un composé de formule I dans laquelle X est un oxygène, l'étape qui comprend la réaction d'un 4-(N-protégé)amino-5-[[(1,1-diméthyléthyl) diméthylsilyl)oxy]-2-oxopentanoate d'alkyle en $C_1$-$C_6$ avec un acide minéral pour former un 4-(N-protégé)amino-tétrahydro-2-hydroxy-2-furannecarboxylate d'alkyle en $C_1$-$C_6$, ladite réaction étant effectuée à la température ordinaire.

9. Dans un procédé selon la revendication 1 pour préparer un composé de formule I dans laquelle X est un oxygène, l'étape qui comprend la réaction d'un 4-(N-protégé)amino-tétrahydro-2-hydroxy-2-furannecarboxylate d'alkyle en $C_1$-$C_6$ avec un agent déshydratant pour obtenir un 4-(N-protégé)amino-4,5-dihydro-2-furannecarboxylate d'alkyle en $C_1$-$C_6$.

10. Procédé selon la revendication 9, dans lequel l'agent déshydratant est le chlorure de thionyle et la réaction est effectuée en présence de pyridine.

11. Procédé selon les revendications 4, 5, 8 ou 9, dans lequel le groupe alkyle en $C_1$-$C_6$ est un groupe méthyle.

12. Dans un procédé selon la revendication 1 pour préparer un composé de formule I dans laquelle X représente un soufre, l'étape qui comprend la déshydratation d'un 4-(N-protégé)amino-tétrahydro-3-hydroxy-2-thiophènecarboxylate d'alkyle en $C_1$-$C_6$.

13. Un procédé selon la revendication 12, dans lequel la déshydratation est effectuée en présence de chlorure de mésyle.

14. Dans un procédé selon les revendications 1 et 12 pour préparer un composé de formule I dans laquelle R représente un soufre, l'étape comprenant la préparation d'un 4-(N-protégé)amino-tétrahydro-3-hydroxy-2-thiophènecarboxylate d'alkyle en $C_1$-$C_6$ par réduction d'un 4-(N-protégé)amino-tétrahydro-3-oxo-2-thiophènecarboxylate d'alkyle en $C_1$-$C_6$ en présence de borohydrure de sodium.

**15.** Procédé selon la revendication 14, dans lequel le 4-(N-protégé)amino-tétrahydro-3-hydroxy-2-thiophènecarboxylate d'alkyle en $C_1$-$C_6$ est obtenu par traitement d'un 4-(N-protégé)amino-3-alcoxy-3-silyloxy-tétrahydro-2-thiophènecarboxylate d'alkyle en $C_1$-$C_6$ avec HF en présence de fluorure de tétrabutylammonium.

**16.** Procédé selon les revendications 1, 4, 5, 8, 9, 12, 14 ou 15, dans lequel le groupe N-protecteur est tert-butoxycarbonyle.

**17.** Procédé selon les revendications 12, 14 ou 15, dans lequel le groupe alkyle en $C_1$-$C_6$ est un groupe éthyle.

**Patentansprüche**
**Patentansprüche für folgende Vertragsstaaten : BE, CH, DE, FR, GB, IT, LU, NL, SE**

**1.** Verbindung der Formel

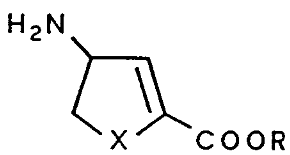

einschließlich der einzelnen Enantiomere und racemischen Gemische davon und deren pharmazeutisch verträgliche Salze, in der R ein Wasserstoffatom oder einen ($C_1$-$C_6$)-Alkylrest bedeutet, und X ein Sauerstoff- oder Schwefelatom ist.

**2.** Verbindung gemäß Anspruch 1, in der X ein Sauerstoffatom ist.

**3.** Verbindung gemäß Anspruch 1, in der X ein Schwefelatom ist.

**4.** Verbindung gemäß den Ansprüchen 2 und 3, in der R ein Wasserstoffatom ist.

**5.** Das (S)-Enantiomer einer Verbindung gemäß Anspruch 2, wobei die Verbindung (S)-4-Amino-4,5-dihydro-2-furancarbonsäure ist.

**6.** Verbindung gemäß Anspruch 3, in der R ein Wasserstoffatom bedeutet, wobei die Verbindung 4-Amino-4,5-dihydro-2-thiophencarbonsäure ist.

**7.** Verfahren zur Herstellung von 4-Amino-4,5-dihydro-2-furancarbonsäure, umfassend die Umsetzung von 4-[[(1,1-Dimethylethoxy)carbonyl]amino]-4,5-dihydro-2-furancarbonsäure mit Trifluoressigsäure unter inerter Atmosphäre.

**8.** Verfahren gemäß Anspruch 7, in dem das Inertgas Argon ist, und die Umsetzung bei Temperaturen im Bereich von etwa 0° bis 10° C durchgeführt wird.

**9.** Verfahren gemäß Anspruch 7, in dem die Trifluoressigsäure rein ist.

**10.** Der Schritt, umfassend die Umsetzung von Methyl-4-[[(1,1-dimethylethyl)dimethylsilyl]oxy]-2-oxovalerianat und Äquivalenten davon mit einer Mineralsäure zur Bildung von Methyl-4-[[(1,1-dimethylethoxy)-carbonyl]amino]tetrahydro-2-hydroxy-2-furancarboxy lat in dem Verfahren zur Herstellung der 4-Amino-4,5-dihydro-2-furancarbonsäure und der Niederalkylester davon gemäß Anspruch 7, wobei die Umsetzung bei etwa Raumtemperatur durchgeführt wird.

**11.** Verfahren gemäß Anspruch 10, in dem das entstandene Produkt mit einem Dehydratisierungsmittel zur Herstellung von Methyl-4-[[(1,1-dimethylethoxy)carbonyl]amino]-4,5-dihydro-2-furancarboxylat umgesetzt wird.

**12.** Verfahren gemäß Anspruch 11, in dem das Dehydratisierungsmittel Thionylchlorid ist, und die Umsetzung in Pyridin durchgeführt wird.

**13.** Verfahren zur Herstellung von 4-Amino-4,5-dihydro-2-thiophencarbonsäure und der $C_{1-6}$-Alkylester davon, umfassend die Abspaltung der Schutzgruppe von einer N-geschützten 4-Amino-4,5-dihydro-2-thiophencarbonsäure und gegebenenfalls Veresterung der Säure durch allgemein übliche Schutzgruppenabspaltungs- und Veresterungsverfahren.

**14.** Verfahren zur Herstellung von 4-Amino-4,5-dihydro-2-thiophencarbonsäure und der $C_{1-6}$-Alkylester davon, umfassend chemische Reduktion und Dehydratisierung eines 3-Hydroxy- oder eines 3-Oxoderivates einer N-geschützten 4-Amino-4,5-dihydro-2-thiophencarbonsäure oder Estern davon und gegebenenfalls Veresterung oder Verseifung der entstandenen Produkte durch allgemein übliche Verfahren.

**15.** Verfahren gemäß Anspruch 14, in dem das chemische Reduktionsmittel Natriumborhydrid und das Dehydratisierungsmittel Mesylchlorid ist.

**16.** Verfahren gemäß den Ansprüchen 13 und 14, in dem die N-Schutzgruppe eine t-Butoxycarbonylgruppe ist.

**Patentansprüche für folgenden Vertragsstaat : AT**

**1.** Verfahren zur Herstellung einer Verbindung der Formel I

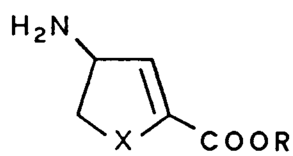

I

einschließlich der einzelnen Enantiomere und racemischen Gemische davon und deren pharmazeutisch verträgliche Salze, in der R ein Wasserstoffatom oder einen ($C_1$-$C_6$)-Niederalkylrest bedeutet, und X ein Sauerstoff- oder Schwefelatom ist, umfassend:

a) Abspaltung der Schutzgruppe von einer N-geschützten 4-Amino-4,5-dihydro-2-furancarbonsäure oder von einem ($C_1$-$C_6$)-Alkylester davon durch Umsetzung unter sauren Bedingungen, falls eine Verbindung der Formel I, in der X ein Sauerstoffatom bedeutet, gewünscht wird,

b) Abspaltung der Schutzgruppe von einer N-geschützten 4-Amino-4,5-dihydro-2-thiophencarbonsäure oder von einem ($C_1$-$C_6$)-Niederalkylester davon durch Umsetzung unter sauren Bedingungen, falls eine Verbindung der Formel I, in der X ein Schwefelatom bedeutet, gewünscht wird,

c) gegebenenfalls Umwandlung einer Verbindung der Formel I, in der R ein Wasserstoffatom bedeutet, durch Veresterungsverfahren in den entsprechenden ($C_1$-$C_6$)-Alkylester, falls die Verbindung gemäß Schritt a) oder b) erhalten wird oder gegebenenfalls Umwandlung der Verbindung in einen weiteren ($C_1$-$C_6$)-Niederalkylester durch Umesterungsverfahren, falls ein Niederalkylester gemäß Schritt a) oder b) erhalten wird.

**2.** Verfahren gemäß Anspruch 1 zur Herstellung einer Verbindung der Formel I, in der R ein Wasserstoffatom ist.

**3.** Verfahren zur Herstellung einer Verbindung gemäß Anspruch 1, in der X ein Sauerstoffatom ist, so charakterisiert, daß ein ($C_1$-$C_6$)-Alkyl-4-azido-4,5-dihydro-2-furancarboxylat reduziert wird, wobei das entsprechende ($C_1$-$C_6$)-Alkyl-4-amino-4,5-dihydro-2-furancarboxylat entsteht.

**4.** Verfahren gemäß Anspruch 1 zur Herstellung einer Verbindung der Formel I, in der X ein Sauerstoffatom bedeutet, in einer racemischen Form, umfassend

a) Umwandlung eines ($C_1$-$C_6$)-Alkyltetrahydro-3-iod-4-isocyanato-2-furancarboxylats in das entsprechende Carbamat durch Umsetzung mit einem Alkohol,

b) Überführung des erhaltenen ($C_1$-$C_6$)-Alkyltetrahydro-3-iod-4-carbamat-2-furancarboxylats in das entsprechende ($C_1$-$C_6$)-Alkyl-4,5-dihydro-4-carbamat-2-furancarboxylat durch Umsetzung mit Diazobicyclooctan in Aceton,

c) Hydrolyse der Alkylesterbindung, wobei sich die entsprechende freie Säure ergibt, und

d) Säurehydrolyse der so erhaltenen 4,5-Dihydro-4-carbamat-2-furancarbonsäure, wobei das entsprechende Aminderivat der Formel I entsteht.

**5.** Verfahren gemäß Anspruch 1 zur Herstellung einer Verbindung, in der X ein Sauerstoffatom ist, so charakterisiert, daß die Abspaltung der Schutzgruppe von einer N-geschützten 4-Amino-4,5-dihydro-2-furancarbonsäure mit Hilfe von Trifluoressigsäure unter inerter Atmosphäre erreicht wird.

**6.** Verfahren gemäß Anspruch 5, in dem das Inertgas Argon ist, und die Umsetzung bei Temperaturen zwischen 0° und 10° C durchgeführt wird.

**7.** Verfahren gemäß Anspruch 5, in dem die Trifluoressigsäure rein ist.

**8.** Der Schritt, umfassend die Umsetzung eines ($C_1$-$C_6$)-Alkyl-4-(N-geschützten)amino-5-[[(1,1-dimethylethyl)dimethylsilyl]oxy]-2-oxovalerianats mit einer Mineralsäure zur Bildung eines ($C_1$-$C_6$)-Alkyl-4-(N-geschützten)aminotetrahydro-2-hydroxy-2-furancarboxylats, in einem Verfahren gemäß Anspruch 1 zur Herstellung einer Verbindung der Formel I, in der X ein Sauerstoffatom bedeutet, wobei die Umsetzung bei Raumtemperatur durchgeführt wird.

**9.** Der Schritt, umfassend die Umsetzung eines ($C_1$-$C_6$)-Alkyl-4-(N-geschützten)aminotetrahydro-2-hydroxy-2-furancarboxylats mit einem Dehydratisierungsmittel, wobei ein ($C_1$-$C_6$)-Alkyl-4-(N-geschütztes)amino-4,5-dihydro-2-furancarboxylat entsteht, in einem Verfahren gemäß Anspruch 1 zur Herstellung einer Verbindung der Formel I, in der X ein Sauerstoffatom ist.

**10.** Verfahren gemäß Anspruch 9, in dem das Dehydratisierungsmittel Thionylchlorid ist, und die Umsetzung in Gegenwart von Pyridin durchgeführt wird.

**11.** Verfahren gemäß den Ansprüchen 4, 5, 8 oder 9, in dem der ($C_1$-$C_6$)-Alkylrest ein Methylrest ist.

**12.** Der Schritt, umfassend die Dehydratisierung eines ($C_1$- $C_6$)-Alkyl-4-(N-geschützten)aminotetrahydro-3-hydroxy-2-thiophencarboxylats, in einem Verfahren gemäß Anspruch 1 zur Herstellung einer Verbindung der Formel I, in der X ein Schwefelatom ist.

**13.** Verfahren gemäß Anspruch 12, in dem die Dehydratisierung in Gegenwart von Mesylchlorid durchgeführt wird.

**14.** Der Schritt, umfassend die Herstellung eines ($C_1$-$C_6$)-Alkyl-4-(N-geschützten)aminotetrahydro-3-hydroxy-2-thiophencarboxylatsdurch Reduktion eines ($C_1$-$C_6$)-Alkyl-4-(N-geschützten)aminotetrahydro-3-oxo-2-thiophencarboxylats in Gegenwart von Natriumborhydrid, in einem Verfahren gemäß den Ansprüchen 1 und 12 zur Herstellung einer Verbindung der Formel I, in der R ein Schwefelatom bedeutet.

**15.** Verfahren gemäß Anspruch 14, in dem ($C_1$-$C_6$)-Alkyl-4-(N-geschütztes)aminotetrahydro-3-hydroxy-2-thiophen durch Behandlung eines ($C_1$-$C_6$)-Alkyl-4-(N-geschützten)amino-3-alkoxy-3-silyloxytetrahydro-2-thiophencarboxylats mit HF in Gegenwart von Tetrabutylammoniumfluorid erhalten wird.

**16.** Verfahren gemäß den Ansprüchen 1, 4, 5, 8, 9, 12, 14 oder 15, in dem die N-Schutzgruppe eine t-Butoxycarbonylgruppe ist.

**17.** Verfahren gemäß den Ansprüchen 12, 14 oder 15, in dem der ($C_1$-$C_6$)-Alkylrest ein Ethylrest ist.